# EUROPEAN PATENT APPLICATION

(11) **EP 4 098 268 A1**
(43) Date of publication of application: **07.12.2022**
(21) Application number: 21177040.9
(22) Date of filing: 01.06.2021
(51) Int. Cl.: A61K 35/37, A61P 1/12

(54) **MODIFICATION OF FECAL MICROBIOTA WITH CAPSULES CONTAINING LYOPHILIZED FECAL MATTER FILTRATE**

(71) Applicant: Lietuvos Sveikatos Mokslu Universitetas, 44307 Kaunas (LT)
(72) Inventor: Kupcinskas, Juozas, 44307 Kaunas (LT); Skieceviciene, Jurgita, 44307 Kaunas (LT); Bernatoniene, Jurga, 44307 Kaunas (LT); Kiudelis, Vytautas, 44307 Kaunas (LT); Petkevicius, Vytenis, 44307 Kaunas (LT); Ivanauskas, Liudas, 44307 Kaunas (LT)
(74) Representative: Zaboliene, Reda

(57) **Abstract**

We provide a method of preparing an oral capsule containing lyophilized donor fecal matter filtrate with depleted microbiota. The capsule may be used for microbiota modification of a patient for the treatment of various diseases or pathological conditions, where such an intervention is indicated, and the patient has a higher risk of increased intestinal barrier permeability. We also provide results of metabolite analysis and comparison between fecal filtrate and lyophilized fecal filtrate.

## Description

### FIELD OF THE INVENTION

The invention relates to oral administration capsules for fecal microbiota modification. Presented capsules contain bacteria-free lyophilized fecal matter that preserves gut microbiota metabolites that is intended to be used for the treatment of gastrointestinal disorders.

### BACKGROUND OF THE INVENTION

Microbiota of the human intestinal tract has been the main focus of a large number of studies during the last two decades. During that time, a great deal of information has been gathered on the role of microbiota in human health and disease. One of the main ways that microbiota affects the host organism is through metabolites - small molecules, that are synthesized as a product or byproduct of microbial metabolism. Bacterial metabolites have a large impact on human physiology, including the intestinal immune system, intestinal mucosal permeability, human energy metabolism.

Currently, the only effective method of intestinal microbiota modification in clinical practice is fecal microbiota transplantation (FMT). During this procedure, the recipient receives FMT solution which contains unmodified donor fecal matter with its entire microbial contents, thus there is a small possibility of transmitting pathogenic or antibiotic resistant bacteria. By using filters to remove bacteria from an FMT suspension, it is possible to minimize the risk of potential pathogen transmission, while retaining the beneficial effects of metabolites to the recipients and their intestinal microenvironment.

Using oral capsules as a means of delivery results in better availability, tolerability and reduced risk and cost when compared to more invasive means of microbiota modification (via colonoscopy or nasoenteric tube). Using oral capsules to deliver FMT solution is a proven treatment method and widely used in clinical practice.

A patent application CN112089736A describes capsules with lyophilized and pulverized fecal microbiota capsules and therefore is similar to present invention. However, the capsule of present invention is safer because it contains bacteria-free fecal matter and prevents the transfer of pathogenic bacteria that can be contained within capsules.

AU2018250206A1 describes capsules with bacteria-free, lyophilized and pulverized fecal matter to treat Parkinson's disease (PD). This invention is similar to present invention due to similar capsule preparation method, however, present invention relates to treatment of gastrointestinal (GI) diseases.

Compared with available technology to treat GI disorders the proposed invention eliminates the need of invasive procedures and reduces the risk of pathogen transmission.

### SUMMARY OF THE INVENTION

The main objective of the present invention is to provide a capsule and method of the preparation of capsule for use in the treatment or prophylaxis of various gastrointestinal diseases or pathological conditions, specifically Clostridioides difficile colitis, antibiotic associated diarrhea, irritable bowel syndrome, functional constipation, small intestinal intestinal overgrowth and inflammatory bowel diseases (ulcerative colitis and Crohn's disease) that is based on oral administration of the capsules containing bacteria-free, lyophilized fecal matter.

Present invention relates to oral administration capsules containing lyophilized bacteria-free fecal matter filtrate containing the metabolites in various combinations specifically for use in the treatment of the GI diseases by modulation of microbiota in a subject in need thereof.

The outer layer of capsule described is made from enteric coating to avoid release in the stomach.

Lyophilization process referred in the present invention is performed using a laboratory freeze-dryer.

Filtrate described in the present invention in prepared by is progressively filtrating the solution containing fecal matter through smaller filter pores up to 0.2 mm to remove microbiota. The filtration is performed by using a 6-15 mm, 0,4-0,8 mm and 0,2 mm filters sequentially. It is important to highlight that this filtrate is bacteria-free filtrate and therefore reduces the risk to transfer pathogens. The filtration is performed using air pressure filtration at a pressure of 1.5-2 bars.

This invention relates to pulverized fecal matter filtrate meaning that capsules contain powder material that contains microbiota metabolites.

The oral microbiota therapeutic composition is prepared for use in the treatment and prophylaxis of various gastrointestinal diseases or pathological conditions selected from Clostridioides difficile colitis, antibiotic associated diarrhea, irritable bowel syndrome, functional constipation, small intestinal intestinal overgrowth and inflammatory bowel diseases, such as ulcerative colitis and Crohn's disease.

### BRIEF DESCRIPTION OF DRAWINGS

Figure 1. Image the lyophilized of microbiota filtrate.
Figure 2. Picture of capsules containing lyophilized fecal matter filtrate.

### DETAILED DESCRIPTION OF THE INVENTION

The present invention relates to a capsule, containing lyophilized donor fecal matter filtrate, that is intended to be used orally for the purposes of treatment or prophylaxis of various gastrointestinal diseases or pathological conditions, specifically Clostridioides difficile colitis, antibiotic associated diarrhea, irritable bowel syndrome, functional constipation, small intestinal intestinal overgrowth and inflammatory bowel diseases (ulcerative colitis and Crohn's disease).

An experiment shows that the metabolic profiles between the solution of the filtered fecal matter and the lyophilized donor fecal matter filtrate were almost identical. The following gut microbiome associated metabolites were identified: (i) short chain fatty acids: *Butanoic acid* (also referred to as Butyric acid; lyophilized sample: 11.41%; filtrated sample: 16.04%), *Acetic acid* (lyophilized sample: 2.15%; filtrated sample: 1.22%), *Pentanoic acid* (also referred to as Valeric Acid; lyophilized sample: 3.33%; filtrated sample: 4.22%), *Glycolic acid* (also referred to as lactic acid; lyophilized sample: 0.98%; filtrated sample: 0.98%); (ii) carboxylic acid *Oxalic acid* (lyophilized sample: 2.37%; filtrated sample: 2.79%); (iii) glycine-conjugated microbial metabolite *2-phenylacetic acid* (lyophilized sample: 6.73%; filtrated sample: 3.25%); (iv) essential amino acids: branched-chain amino acid *isoleucine* (lyophilized sample: 0.46%; filtrated sample: 0.70%), it's optical isomer *L-isoleucine* (lyophilized sample: 0.71%; filtrated sample: 1%)) and *L-Glutamic acid* (also referred to as glutamate (the anion); lyophilized sample: 1.61%; filtrated sample: 2.75%).

### Donor selection

The fecal matter is produced from stool that is donated by healthy volunteers. Before becoming donors, the volunteers have to sign an informed consent form and undergo the selection process. The process starts from a medical interview, which aims to assess the donor's risk of infections and past interventions that might have altered the gut microbiota. After that, blood and stool tests for potentially transmissible infectious diseases are performed. After passing the selection process, the volunteer may start donating stool, which is processed according to a standardized protocol, frozen in -80°C and stored until use. The entire donor selection and testing process, the processing and banking of the stool samples is performed according to international recommendations.

### Stool preparation

Donor stool was collected and stored at 4°C for up to 3 hours until processing (depending on the time of delivery). Approximately 50 g of donor stool was processed using a commercial blender and mixed with 350 ml of sterile saline (0,9 % sodium chloride). The resulting solution was filtered 2 times using a double and triple gauze filter. Afterwards, the solution was centrifuged at 1900 RCF for 20 min at room temperature and then filtered 2 - 3 times using a coffee filter. This process results in 350 ml of brown, turbid liquid.

### Filtration and lyophilization of the fecal matter

The prepared stool was then filtered using a custom-built air pressure filtration system at a pressure of 1.5-2 bars. Small particles were removed using a 6-15 mm filter and then a 0,4-0,8 mm filter. Afterwards, a 0,2 mm filtration unit was used to remove microbiota. The resulting filtrate was a clear, light brown colored liquid.

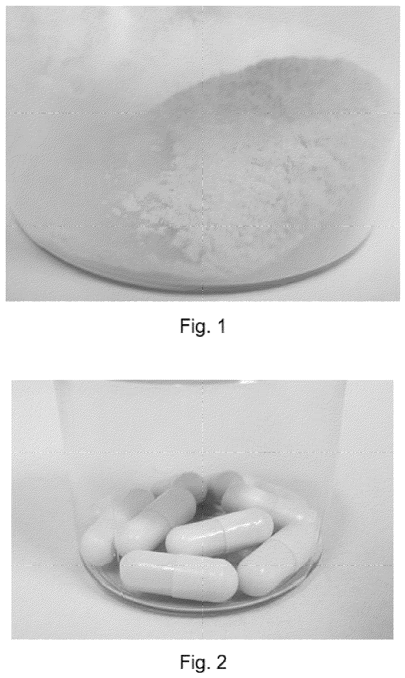

Lyophilization was performed using a laboratory freeze-dryer. The condenser is turned on -30 min before lyophilization to reach the temperature of -53°C. The fecal matter filtrate was frozen at -50°C 24 hours before the procedure. Lyophilization takes 48 hours to complete, and the resulting mass is pulverized into sand-colored powder (Fig. 1).

### Capsule preparation

The lyophilized fecal filtrate is placed in single-layered capsules with enteric coating to avoid release in the stomach (Fig. 2). 35 capsules are manufactured out of the 50 g of pure donor fecal matter, with each capsule containing 0,056 g of lyophilized fecal matter filtrate.

### Metabolome analysis of filtrated fecal sample and lyophilized filtrate

Sample derivatization procedure: 0.5 ml of prepared extract solution was evaporated to dryness with a stream of nitrogen gas. Dried extract sample was diluted into 0.1 mL of extraction solvent (acetonitrile) and 0.1 mL derivatization agent (MTBSTFA) was added in sequence. The vial was sealed and oscillated by vortex-mixer for 1 min, and then, to allow the mixture to react it was placed in glycerol bath at 100 °C for 150 min. The subsequent solution was transferred to 200 µL insert placed autosampler vials, and 3 µL aliquot was injected into GC-MS system for analysis.

GC/MS method: Analyses were performed using a SHIMADZU GC/MS-QP2010nc (Shimadzu Technologies). A robotic autosampler and a split/splitless injection port were used. Injection port temperature was kept at 260 °C until the end of analysis. The separation of analytes was carried out on a with Rxi-5 ms (Restek Corporation capillary column (30 m long, 0.25 mm outer diameter and 0.25 µm liquid - stationary phase thickness) with a liquid stationary phase) 5% diphenyl and 95% polydimethylsiloxane) with helium at a purity of 99.999% as the carrier gas in a constant flow of 1.49 mL/min. The oven temperature was programmed at 75 °C for 5 min, then increased to 290 °C at 10 °C/min and increased to 320 °C at 20 °C/min and kept for 10 min. Total time was 41 min. The temperatures of the MS interface and ion source were set at 280, 200 °C, respectively. The split mode was used for analysis, the ratio of split was 10. The MS was operated in positive mode (electron energy 70 eV). For compound identification we used GC/MS libraries NIST14, NIST14s, WR10, WR10R. Full-scan acquisition was performed with the mass detection range set at 35 to 500 m/z. Data acquisition and analysis were executed by LabSolution GC/MS (version 5.71) (Shimadzu Corporation).

## Claims

1. An oral microbiota therapeutic composition comprising:
capsule;
lyophilized fecal matter filtrate;
for use in the treatment or prevention of gastrointestinal diseases or pathological conditions by modulation of microbiota in a subject in need thereof.

2. The oral microbiota therapeutic composition for use according to claim 1, wherein said therapeutic composition comprises bacteria-free lyophilized fecal matter.

3. The oral microbiota therapeutic composition for use according to claim 1, wherein bacteria-free lyophilized fecal matter is filtrate prepared by using a 6-15 mm, 0,4-0,8 mm and 0,2 mm filters sequentially.

4. The oral microbiota therapeutic composition for use according to claim 3, wherein bacteria-free lyophilized fecal filtrate is prepared using air pressure filtration.

5. The oral microbiota therapeutic composition for use according to claim 3 or 4, wherein filtration is performed at a pressure of 1.5-2 bars.

6. The oral microbiota therapeutic composition for use according to claim 1, wherein said composition is formulated as enteric coated capsule.

7. The oral microbiota therapeutic composition for use according to any of claims 1-6, for use in the treatment and prophylaxis of various gastrointestinal diseases or pathological conditions selected from Clostridioides difficile colitis, antibiotic associated diarrhea, irritable bowel syndrome, functional constipation, small intestinal intestinal overgrowth and inflammatory bowel diseases, such as ulcerative colitis and Crohn's disease.
